Europäisches Patentamt

European Patent Office

Office européen des brevets

(11) Veröffentlichungsnummer: **0 269 007**

**A1**

(12) # EUROPÄISCHE PATENTANMELDUNG

(21) Anmeldenummer: **87117105.4**

(22) Anmeldetag: **19.11.87**

(51) Int. Cl.⁴ **A61N 1/36 , C04B 37/02 , B23K 35/30 , H01B 17/30**

(30) Priorität: **28.11.86 DE 3640639**

(43) Veröffentlichungstag der Anmeldung:
**01.06.88 Patentblatt 88/22**

(84) Benannte Vertragsstaaten:
**DE FR GB IT SE**

(71) Anmelder: **Siemens Aktiengesellschaft Berlin und München**
**Wittelsbacherplatz 2**
**D-8000 München 2(DE)**

(72) Erfinder: **Hübner, Erwin, Dr. rer. nat.**

**Verstorben(DE)**

(54) **Keramik-Metall-Durchführung, insbesondere für Nerven- oder Herzschrittmacher, und Verfahren zu deren Herstellung.**

(57) Die Erfindung bezieht sich auf eine Keramik-Metall-Durchführung, insbesondere für Nerven-oder Herzschrittmacher, mit einer mindestens eine Elektrodendurchführung aufweisenden Scheibe (4) aus hochreiner Keramik, die über einen Lotspalt (1) in einen Flanschring (2) aus einem insbesondere gegenüber Körperelektrolyten resistenten Metall wie Titan oder Tantal vakuumdicht eingelötet ist, wobei die thermischen Ausdehnungskoeffizienten des Materials der Verbindungspartner nahezu einander angepaßt sind.

Bei dieser Keramik-Metall-Durchführung sollen Anrisse in der Keramikscheibe (4) im Bereich der Lotverbindung (3) vermieden werden. Die Erfindung sieht hierzu vor, daß der Lotspalt (1) eine solche Breite aufweist, daß bei Löttemperatur in der sich bildenden Lotbrücke der Diffusionsweg des Metalls des Flanschringes (2) in die Lotverbindung (3) eine gewisse Strecke nicht überschreiten kann, so daß um die Keramikscheibe (4) herum eine Lotzone gebildet ist, die frei vom Metall des Flanschringes (2) ist und dadurch ihre Duktilität behält.

Eine erfindungsgemäße Keramik-Metall-Durchführung findet insbesondere bei Nerven-und Herzschrittmachern Anwendung.

## Keramik-Metall-Durchführung, insbesondere für Nerven-oder Herzschrittmacher, und Verfahren zu deren Herstellung.

Die Erfindung bezieht sich auf eine Keramik-Metall-Durchführung nach dem Oberbegriff des Anspruchs 1.

Nerven-bzw. Herzschrittmacher bestehen im wesentlichen aus vier Bauelementen, einer elektronischen Schaltung zur Erzeugung der Stimulationsimpulse und zur Verarbeitung von Steuersignalen, einer Batterie als Energiequelle, einer hermetisch dichten Einkapselung, die Schaltung und Energiequelle vor Körperelektrolyten schützt, sowie aus Elektroden, die aus einer dauerwechsellastfesten, elektrisch gegen den Körper isolierten Zuleitung und den eigentlichen Elektrodenkopf bestehen und die Verbindung zu den Reizleitern herstellen.

Bei der Herstellung derartiger für eine Körperimplantation geeigneter Geräte ist insbesondere bei der Herstellung der Keramik-Durchführung für die Elektroden dafür Sorge zu tragen, daß Materialien verwendet werden, die gegenüber Körperelektrolyten resistent sind und daß die Durchführung vakuumdicht und damit auch feuchtigkeitsundurchlässig ist. Von besonderem Nachteil ist bei derartigen Durchführungen ein eventuelles Undichtwerden, das beim vakuumdichten Verlöten der Metall-und Keramikteile durch Rißbildung in der Keramik hervorgerufen wird.

Alle bisher bekannten Konstruktionen von Keramik-Metall-Durchführungen gehen von der Überlegung aus, daß beim Verlöten von im Ausdehnungskoeffizienten angepaßten Verbindungspartnern diese auch konstruktiv gut aneinander angepaßt sein müssen. Daher weisen die bekannten Durchführungen an den Lötstellen auch nur kleine Spalte auf, die verhältnismäßig leicht mit Lot zu füllen sind. Bei der Verlötung eines Titanflansches und einer Keramikperle mittels Gold diffundiert jedoch Titan in das Gold und umgekehrt.

Die entstehende Lotverbindung ist sehr spröde und hart. Da die Ausdehnung des Titans beim Lotschmelzpunkt um einiges größer ist als die der Keramik, zieht es sich beim Abkühlen auch mehr zusammen als die Keramik. Als Folge davon wird auf die Keramik ein Zug ausgeübt, der zur Ausbildung von Sprüngen in der Keramik führt, da deren Zugfestigkeit relativ gering ist. Eine Aufnahme der Zugkräfte ist aber bei der nun spröden Lotverbindung nicht mehr möglich.

Der Erfindung liegt die Aufgabe zugrunde, diese Nachteile zu vermeiden und eine Keramik-Metall-Durchführung zu schaffen, bei der Anrisse in der Keramik vermieden werden, so daß diese Durchführung hermetisch dicht ist und sich insbesondere für Geräte eignet, die zur Implantation im menschlichen Körper vorgesehen sind.

Diese Aufgabe wird erfindungsgemäß durch eine Keramik-Metall-Durchführung mit den Merkmalen des Anspruchs 1 gelöst.

Die hochreine Keramik besteht dabei vorzugsweise aus Aluminiumoxid (Aluminiumoxidanteil größer als 98%) und das Lot aus Gold.

Die Breite des Lotspaltes beträgt zweckmäßig ungefähr 1% bis 3% des Durchmessers der Keramikscheibe.

Die erfindungsgemäße Keramik-Metall-Durchführung wird vorzugsweise folgendermaßen hergestellt.

Eine Keramikscheibe wird im Lötbereich mit einer Metallisierung, zweckmäßig aus Titanhydrid, versehen. Diese Scheibe ist so bemessen, daß sie mit dem Flanschring, der zweckmäßig aus Titan oder Tantal besteht, in diesen eingefügt einen relativ breiten Lotspalt bildet. Auf den Flanschkragen wird dann ein Golddraht aufgelegt und diese Anordnung anschließend im Vakuum auf die Schmelztemperatur des als Lot verwendeten Goldes erhitzt. Die Haltezeit dieser Temperatur wird so kurz gehalten, zweckmäßig 1min. bis 1,5min, daß ein Hindurchdiffundieren bzw. Durchlegieren des Titans oder Tantals durch die entstehende, relativ lange Lotverbindung (Lotbrücke) verhindert wird, so daß eine Zone nahezu reinen Goldes zur Keramikscheibe hin erhalten wird. Voraussetzung für das Gelingen solcher Lötungen sind zentrierende Lötlehren oder die Verwendung von selbstzentrierenden Flanschen.

Die mit der Erfindung erzielten Vorteile bestehen insbesondere darin, daß entgegen der allgemein vertretenen Auffassung durch Verbreiterung des Lotspaltes zwischen Flanschring und Keramikscheibe und damit "Verschlechterung" der geometrischen Anpassung der zu verlötenden Teile erreicht wird, daß ein Bilden von Anrissen und damit Undichtwerden der Keramik vermieden wird. Das wird dadurch erreicht, daß neben der Verbreiterung des Lotspaltes die Haltezeit der Temperatur des Goldschmelzpunktes während des Lötens nur sehr kurz gehalten wird, so daß ein "Durchlegieren" der entstandenen nunmehr verhältnismäßig langen Lotbrücke nicht mehr möglich ist. Es bleibt in der Lotbrücke also eine Zone reinen Goldes und damit eine gewisse Duktilität der Lotverbindung übrig. In dieser werden die natürlich auch bei dieser Keramik-Metall-Durchführung auftretenden Zugkräfte aufgefangen und wirken nicht mehr zugbelastend auf die Keramik.

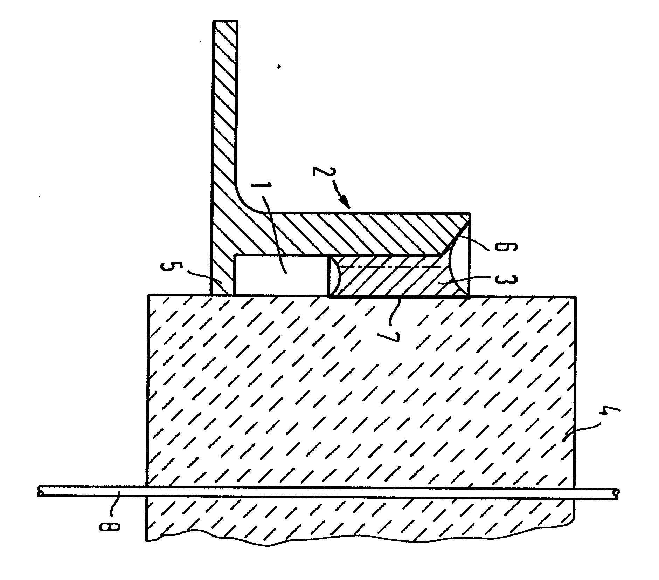

Anhand eines in der Figur dargestellten, bevorzugten Ausführungsbeispiels wird die Erfindung näher erläutert. Die Figur zeigt schematisch einen Ausschnitt einer erfindungsgemäßen Keramik-Metall-Durchführung.

Die Keramik-Metall-Durchführung besteht im wesentlichen aus einer Scheibe 4 aus hochreiner Keramik, die über einen Lotspalt 1 in einem Flanschring 2 aus Tantal oder Titan vakuumdicht eingelötet ist. Der Lotspalt 1 hat eine solche Breite, daß bei Löttemperatur, d.h. bei der Schmelztemperatur des als Lot verwendeten Goldes der Diffusionsweg des Metalls des Flanschringes 2 in die sich bildende Lotbrücke bzw. Lotverbindung 3 eine gewisse Strekke nicht überschreiten kann. Das ungefähre Ende der Diffusionsstrecke ist in der Lotverbindung 3 durch eine strichpunktierte Linie angedeutet. Der Bereich zwischen der strichpunktierten Linie und der auf der Keramikscheibe 4 zur besseren Haftung der Lotverbindung 3 vorgesehenen Metallisierung 7 ist frei vom Metall des Flanschringes 2 und besteht aus als Lot vorzugsweise verwendetem Gold. Bei dem in der Figur dargestellten Ausführungsbeispiel weist der Flanschring 2 zur Selbstzentrierung an seinem Innenende einen nach innen gerichteten Vorsprung 5 auf, der die Breite des Lotspaltes 1 bestimmt. Die Lotverbindung 3 endet oberhalb des Vorsprunges 5 im Bereich der Metallisierung 7, so daß im Bereich des Vorsprunges 5 keine durch die Lotverbindung 3 bedingten Zugkräfte auftreten können. Zum leichteren Einbringen des Lotes in den Lotspalt 1 ist es vorteilhaft, das Außenende (Flanschkragen) des Flanschringes 2 mit einer nach innen verlaufenden Abschrägung 6 zu versehen. In diesem Ausführungsbeispiel ist lediglich eine durch die Keramikscheibe 4 vakuumdicht hindurch geführte Elektrodenleitung 8 dargestellt. Je nach Verwendungszweck können jedoch auch mehrere voneinander beabstandet angeordnete Elektrodenleitungen durch die Keramikscheibe 4 hindurchgeführt sein. Als Material für eine solche Elektrodenleitung kommt bevorzugt Platin oder Niob in Betracht.

**Ansprüche**

1. Keramik-Metall-Durchführung, insbesondere für Herz-oder Nervenschrittmacher, mit einer mindestens eine Elektrodendurchführung aufweisenden Scheibe aus hochreiner Keramik, die über einen Lotspalt in einen Flanschring aus einem insbesondere gegenüber Körperelektrolyten resistenten Metall wie Titan oder Tantal vakuumdicht eingelötet ist, wobei die thermischen Ausdehnungskoeffizienten des Materials der Verbindungspartner nahezu angepaßt sind, **dadurch gekennzeichnet**, daß der Lotspalt (1) eine solche Breite aufweist, daß bei Löttemperatur in der sich bildenden Lotbrücke der Diffusionsweg des Metalls des Flanschringes (2) in die Lotverbindung (3) eine gewisse Strecke nicht überschreiten kann, so daß um die Keramikscheibe (4) herum eine Lotzone gebildet ist, die frei vom Metall des Flanschringes (2) ist und dadurch ihre Duktilität behält.

2. Keramik-Metall-Durchführung nach Anspruch 1, **dadurch gekennzeichnet**, daß die hochreine Keramik aus Aluminiumoxid besteht.

3. Keramik-Metall-Durchführung nach Anspruch 1 oder 2, **dadurch gekennzeichnet**, daß das Lot aus Gold besteht.

4. Keramik-Metall-Durchführung nach einem der Ansprüche 1 bis 3, **dadurch gekennzeichnet**, daß der Flanschring (2) an seinem Innenende einen nach innen gerichteten Vorsprung (5) aufweist, der die Breite des Lotspaltes (1) bestimmt und zur Selbstzentrierung dient, und daß die Lotverbindung (3) oberhalb des Vorsprunges (5) endet.

5. Keramik-Metall-Durchführung nach einem der Ansprüche 1 bis 4, **dadurch gekennzeichnet**, daß die Breite des Lotspaltes (1) ungefähr 1% bis 3% des Durchmessers der Keramikscheibe (4) beträgt.

6. Verfahren zum Herstellen einer Keramik-Metall-Durchführung nach einem der Ansprüche 1 bis 5, **dadurch gekennzeichnet**, daß eine im Lötbereich mit einer Metallisierung (7) versehene Keramikscheibe (4) einen relativ breiten Lotspalt (1) bildend in einen Flanschring (2) aus Titan oder Tantal eingefügt und arretiert wird, daß auf den Flanschkragen ein Golddraht aufgelegt wird, daß die Anordnung im Vakuum auf Schmelztemperatur des Goldes erhitzt wird, so daß eine Lotbrücke gebildet wird, daß die Haltezeit der Schmelztemperatur so kurz gehalten wird, daß ein Hindurchdiffundieren (Hindurchlegieren) des Titans oder Tantals durch die entstandene relativ lange Lotverbindung (3) verhindert wird, so daß eine Zone nahezu reinen Goldes zur Keramikscheibe (4) hin erhalten wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 005 312  (MEDTRONIC INC.) <br> * Zusammenfassung; Seite 1, Zeilen 6-23; Seite 3, Zeilen 11-29; Seite 8, Zeilen 4-34; Seite 10, Zeile 6 - Seite 11, Zeile 24; Figuren 1-4 * <br> --- | 1-3,6 | A 61 N 1/36 <br> C 04 B 37/02 <br> B 23 K 35/30 <br> H 01 B 17/30 |
| A | WO-A-8 001 620  (MEDTRONIC INC.) <br> * Zusammenfassung; Seite 1, Zeilen 18-24; Seite 2, Zeilen 14-27; Seite 3, Zeilen 7-21,29-34; Seite 4, Zeile 12 - Seite 5, Zeile 8; Figur 4 * <br> --- | 1-3,6 | |
| A | US-A-4 225 262  (MEDTRONIC INC.) <br> * Zusammenfassung; Spalte 1, Zeilen 24-28,52-58; Spalte 2, Zeilen 9-20,39-64; Figur 1 * <br> --- | 1-3,6 | |
| A | US-A-3 134 230  (R.W. LYNCH) <br> * Spalte 2, Zeilen 40-72; Spalte 3, Zeilen 8-48; Figur 2 * <br> ----- | 2,4 | |

RECHERCHIERTE SACHGEBIETE (Int. Cl.4)

A 61 N
B 23 K
H 01 B

Der vorliegende Recherchenbericht wurde für alle Patentansprüche erstellt

| Recherchenort | Abschlußdatum der Recherche | Prüfer |
|---|---|---|
| DEN HAAG | 15-02-1988 | RIEB K.D. |